# EUROPEAN PATENT APPLICATION

(11) **EP 4 803 636 A1**
(43) Date of publication of application: **09.09.2026**
(21) Application number: 24885793.0
(22) Date of filing: 31.10.2024
(51) Int. Cl.: C12Q 1/6837, C12M 1/34, C12Q 1/686, C12Q 1/6869, G01N 33/50

(54) **MULTI-STAGE INSPECTION METHOD INCLUDING MULTIPLE INSPECTION METHODS, SYSTEM FOR EXECUTING SAID METHOD, AND BILLING SYSTEM**

(30) Priority: 02.11.2023 JP 2023188135
(71) Applicant: Blue Industries Inc., Tokyo 130-0013 (JP)
(72) Inventor: KUJI Tomoaki, Tokyo 103-0013 (JP)
(74) Representative: Marks & Clerk LLP
(86) International application number: PCT/JP2024/038786
(87) International publication number: WO 2025/095026

(57) **Abstract**

The present disclosure provides a method for testing a plurality of biological samples respectively obtained from a plurality of subjects, the method comprising a first step and a second step, and narrowing down subjects to be tested in the second step by the first step, thereby reducing the number of tests in the second step. Thereby, for example, the number of performances of the complicated or expensive second step is reduced, enabling speed-up and/or cost reduction through reduction in testing volume.

## Description

### TECHNICAL FIELD

The present disclosure relates to a multi-step testing method comprising a plurality of testing methods, a system for executing the method, and a billing system.

### BACKGROUND ART

Prediction of a health condition, a disease condition, responsiveness to a drug, or a risk of developing a disease has medical needs. Comprehensive understanding of various gene expressions of a living body leads to more accurate prediction. Transcriptome analysis of a living body has become possible at a realistic cost at a research level while ensuring overwhelming comprehensiveness with the advent of next-generation sequencers (NGS). However, analysis by NGS is still expensive for use as a clinical test. Besides NGS, there are many testing techniques whose procedures are complicated or whose costs are too high for use as a clinical test.

Patent Document 1 discloses a system for selecting a therapeutic drug for cancer equipped with next-generation sequencing and an automated slide staining device. The system disclosed in Patent Document 1 can cut out a specimen, subject it to next-generation sequencing, and subject it to immunohistochemical staining.

### PRIOR ART

### PATENT DOCUMENTS

Patent Document 1: WO2017/132276A

### SUMMARY OF THE INVENTION

The present invention provides a multi-step testing method comprising a plurality of testing methods, and a billing system. Specifically, the present disclosure provides a method that enables speed-up and/or cost reduction of overall testing in testing for a plurality of subjects, a system for executing the method, and a billing system.

According to the present invention, for example, the following inventions may be provided.
(1) A method for testing a plurality of biological samples respectively obtained from a plurality of subjects,
   the method comprising a first step and a second step, the second step being performed after the first step,
   the first step comprising:
      measuring levels of s types {s is a natural number of 1 or more} of a first biomarker contained in each of the biological samples; and identifying a subject from which a biological sample in which a level of the first biomarker satisfies a first criterion is derived,
      the second step comprising:
         measuring levels of t types {t is a natural number of 2 or more, preferably a natural number exceeding s} of a second biomarker contained in each of biological samples obtained from the subject identified in the first step; and identifying a biological sample in which the measured level satisfies a second criterion,
         wherein the number of analyses of biological samples in the first step is p,
         the number of analyses of biological samples in the second step is q,
         the number of biological samples in which the measured level satisfies the second criterion is r, the number of biological samples in which the measured level satisfies the second criterion when all p biological samples are subjected to the second step without performing the first step is r', and r/q is greater than r'/p,
         a biological sample obtained from a subject from which a biological sample in which the measured level does not satisfy the first criterion in the first step is derived is not analyzed in the second step, and thereby, p and q are natural numbers satisfying p > q.
(2) The method according to (1) above, wherein
   the first step comprises measuring presence or absence of s types {s is a natural number of 1 or more} of the first biomarker contained in each of the biological samples; and identifying a subject from which a biological sample in which presence or absence of the first biomarker satisfies the first criterion is derived.
(3) The method according to (1) or (2) above, wherein
   the level of the first biomarker is measured by a PCR method, and
   the level of the second biomarker is measured by a sequencing method.
(4) The method according to (3) above, wherein the PCR method is a digital PCR method.
(5) The method according to (3) or (4) above, wherein t is a natural number of 20 or more.
(6) The method according to any one of (1) to (5) above, wherein
   in the second step, a prediction model constructed so as to be able to predict a health condition of a subject from which a biological sample is derived, from a level of the second biomarker in the biological sample, by causing association information between levels of the second biomarker of each of biological samples obtained from a plurality of subjects measured in advance and health conditions of each of the plurality of subjects to be learned by machine learning, includes the second criterion relating to the level of the second biomarker, and the prediction model evaluates whether the level of the second biomarker in the biological sample satisfies the second criterion.
(7) The method according to any one of (1) to (6) above, further comprising one or more correction cycles of the first criterion,
   the correction cycle of the first criterion comprising:
   associating the level of the first biomarker and the level of the second biomarker of each of the subjects;
   causing the associated level of the first biomarker and the level of the second biomarker to be learned by machine learning, and constructing a prediction model capable of predicting the level of the second biomarker from the level of the first biomarker;
   wherein the number of biological samples in which the measured level satisfies the second criterion is r {r is a natural number equal to or less than q};
   correcting the first criterion so that r becomes larger based on the prediction model without increasing q (that is, while q is constant or preferably decreasing).
(8) A system for testing a plurality of biological samples respectively obtained from a plurality of subjects,
   having one or more processors, a memory, and an output device,
   one or more programs being stored in the memory and configured to be executed by the one or more processors,
   the one or more programs comprising instructions for:
      causing the memory to store levels of s types {s is a natural number of 1 or more} of a first biomarker contained in each of biological samples obtained from the subjects (number of individuals: p) and a first criterion, causing the processor to calculate whether the level of the first biomarker stored in the memory falls under the first criterion, and causing the processor to identify subjects (number of individuals: q) from which biological samples in which the level of the first biomarker falls under the first criterion are derived, and causing the output device to output the subjects (a first step); and
      causing the memory to store levels of t types {t is a natural number of 1 or more} of a second biomarker contained in each of biological samples obtained from the subjects (number of individuals: q) output by the output device and a second criterion, causing the processor to calculate whether the level of the second biomarker stored in the memory falls under the second criterion, and causing the processor to identify subjects (number of individuals: r) from which biological samples in which the level of the second biomarker falls under the second criterion are derived, and causing the output device to output the subjects (a second step),
      wherein p, q, and r are each natural numbers, p > q > r, the number of biological samples in which the measured level satisfies the second criterion when all p biological samples are subjected to the second step is r', and r/q is greater than r'/p.
(9) The system according to (8) above, wherein
   a time required for a program executing the first step and the second step is shorter than a time required for executing a control program,
   the control program comprising instructions for causing the memory to store levels of t types {t is a natural number of 1 or more} of the second biomarker contained in each of biological samples obtained from subjects (number of individuals: p) and the second criterion, causing the processor to calculate whether the level of the second biomarker stored in the memory falls under the second criterion, and causing the processor to identify subjects (number of individuals: r) from which biological samples in which the level of the second biomarker falls under the second criterion are derived, and causing the output device to output the subjects.
(10) The system according to (8) or (9) above, wherein
   the program further comprises instructions for executing one or more correction cycles of the first criterion,
   the correction cycle of the first criterion comprising:
      associating the level of the first biomarker and the level of the second biomarker of each of the subjects;
      causing the associated level of the first biomarker and the level of the second biomarker to be learned by machine learning, and constructing a prediction model capable of predicting the level of the second biomarker from the level of the first biomarker;
      wherein the number of biological samples in which the measured level satisfies the second criterion is r {r is a natural number equal to or less than q};
      correcting the first criterion so that r becomes larger based on the prediction model without increasing q (that is, while q is constant or preferably decreasing).

### BRIEF DESCRIPTION OF THE DRAWINGS

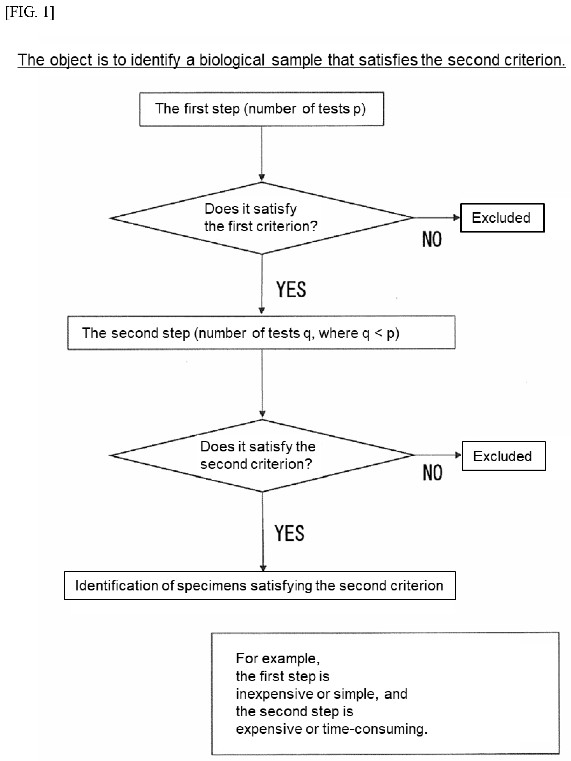

[FIG. 1] FIG. 1 shows the scheme of testing in the multi-step testing method of the present disclosure.

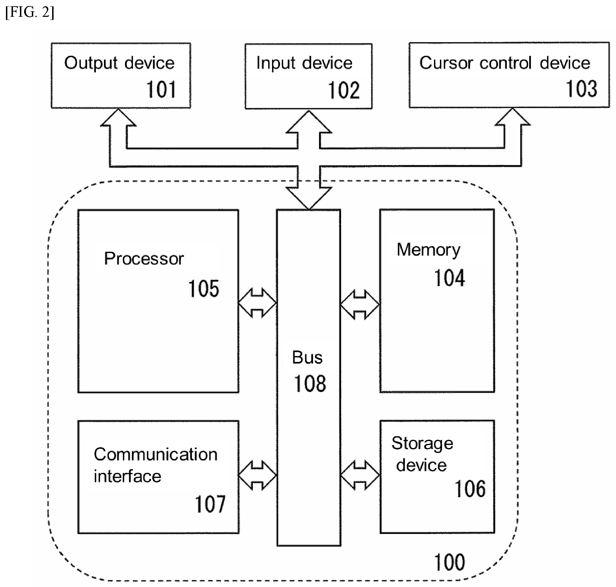

[FIG. 2] FIG. 2 shows a schematic diagram of the testing system of the present disclosure.

### DESCRIPTION OF THE EMBODIMENTS

### <Definition of Terms>

In the present specification, a "subject" may be a mammal, and examples thereof include rodents such as rats and mice, non-rodent mammals such as horses, goats, cattle, pigs, sheep, dogs, and cats, and humans and non-human primates. The plurality of subjects may be of different species, but are preferably of the same species, for example, humans.

In the present specification, a "biological sample" means a sample obtained from a subject (for example, tissue, cells, extracellular fluid, and body fluid). Examples of the body fluid include blood, urine, ascites, pleural effusion, saliva, nasal discharge, and secretions (for example, endocrine fluid and exocrine fluid, for example, digestive fluids such as pancreatic fluid, sweat, and tears). In the present specification, a "blood sample" is blood obtained from a subject (for example, peripheral blood) or a sample derived from blood (for example, serum and plasma). The measurement target specimen in the first step described later is referred to as a first biological sample, and the measurement target specimen in the second step is referred to as a second biological sample. The first biological sample and the second biological sample may be the same or the same type, different (that is, different of the same type), or different types.

In the present specification, a "biomarker" is a factor that is detected in or increases or decreases in a biological sample depending on a health condition, a risk of developing a disease, responsiveness to a drug, and/or a disease condition of a subject. Using the presence or amount of a biomarker as an indicator, it is possible to predict a health condition, a risk of developing a disease, responsiveness to a drug, and/or a disease condition of a subject. Examples of the biomarker include, without being particularly limited, presence or absence of nucleic acids such as DNA and RNA (mRNA and microRNA), epigenetic modification of a genome, the sequence of the nucleic acid, and the amount of the nucleic acid, types and amounts of exosomes, types and amounts of proteins, presence or absence and amounts of post-translational modifications of specific proteins (including presence or absence and amounts of glycosylation modification, phosphorylation, and other modifications), and types and amounts of metabolites.

In the present specification, "comprising" encompasses "consisting of". In addition, the singular form includes the plural form unless otherwise indicated.

### <Method of the Present Disclosure>

According to the present disclosure, a multi-step testing method comprising a plurality of testing methods is provided. The testing method may be an in vivo testing method or an in vitro testing method, and is preferably an in vitro testing method. In an aspect, the testing method of the present invention does not include medical practice (particularly diagnostic practice or surgical procedure). The testing method of the present invention is industrially applicable. The testing method of the present invention may be a method for obtaining preliminary information for estimating or predicting a health condition, a risk of developing a disease, responsiveness to a drug, and/or a disease condition of a subject.

The multi-step testing method comprises a first step and a second step, the second step is performed after the first step, and the first step and the second step are different. Alternatively, the multi-step testing method comprises a first-stage testing method comprising a first step and a second-stage testing method comprising a second step, the second-stage testing method is performed after the first-stage testing method, and the first-stage testing method and the second-stage testing method are different. According to the present disclosure, the first-stage testing method comprising the first step and the second-stage testing method comprising the second step are different. Although a step is a step that can be independently performed, it need only include acquisition of data or analysis of data for obtaining estimation results or prediction results having clinical significance, and it is not necessary to provide estimation results or prediction results having clinical significance, whereas a testing method independently provides results having technical significance or clinical significance. What the first-stage testing method and the second-stage testing method attempt to estimate or predict may be the same, and is preferably the same.

Preferably, the multi-step method comprises a preliminary method comprising a first step and a testing method comprising a second step. The preliminary method comprising the first step need only include acquisition of data or analysis of data for obtaining estimation results or prediction results having clinical significance, and it is not necessary to provide estimation results or prediction results having clinical significance, whereas the testing method independently provides results having technical significance or clinical significance. The preliminary method comprising the first step is a method for narrowing down subjects to be subjected to the second step, and is therefore positioned as a preliminary method. However, the preliminary method comprising the first step may by itself provide estimation results or prediction results having clinical significance. When it is possible to predict a health condition, a risk of developing a disease, responsiveness to a drug, and/or a disease condition of a subject without subjecting to the second step, the second step becomes unnecessary, and when prediction is not possible, it is also possible to apply the second step to the biological sample.

In an aspect, the first testing method and the second testing method predict a health condition, a risk of developing a disease, responsiveness to a drug, and/or a disease condition of a subject. In an aspect, the first testing method and the second testing method predict a health condition, a risk of developing a disease, and/or a disease condition of a subject. In an aspect, in the first step, the preliminary method comprising the first step, and the first testing method, when it is predicted that the second biomarker may satisfy the second criterion in the second testing method, the second biological sample is subjected to the second testing method. In an aspect, in the first step, the preliminary method comprising the first step, and the first testing method, only when it is predicted that the second biomarker will not satisfy the second criterion in the second testing method, the second biological sample is not subjected to the second testing method.

In an aspect, the biological sample in the first step, the preliminary method comprising the first step, the first testing method comprising the first step, and the biological sample in the second testing method comprising the second step are the same or the same type. In an aspect, the biological sample in the first step, the preliminary method comprising the first step, the first testing method comprising the first step, and the biological sample in the second testing method comprising the second step are different or different types.

When the biological sample in the first step, the preliminary method comprising the first step, the first testing method comprising the first step, and the biological sample in the second testing method comprising the second step are the same or the same type, they may be obtained from the subject in a single operation.

When the biological sample in the first step, the preliminary method comprising the first step, the first testing method comprising the first step (first biological sample) and the biological sample in the second testing method comprising the second step (second biological sample) are different or different types, the first biological sample may be collected at the same period as the second biological sample, or may be collected at a different period.

When the first biological sample and the second biological sample are collected at the same period, one package comprising the first biological sample and the second biological sample may be provided, and the package is appropriately transported from the subject to a testing site. Here, "same period" does not need to be completely simultaneous, but is sufficiently close that the first biological sample and the second biological sample can be respectively obtained and included in one package for transport. According to the present disclosure, a biological sample collection kit is provided that comprises a first container for introducing the first biological sample and a second container for introducing the second biological sample. The biological sample collection kit is for carrying out the method of the present disclosure. The first biological sample and the second biological sample collected by the biological sample collection kit are respectively analyzed by the first step and the second step. The first container and the second container can be visually distinguished from each other by color and/or shape. When the first biological sample and the second biological sample are collected at different periods, in the first step, the preliminary method comprising the first step, or the testing method comprising the first step, when analysis of the subject by the second step is desired, the second biological sample may be collected from the subject.

In an aspect, in the first step, the preliminary method comprising the first step, and the first testing method, it is possible to calculate a probability that the subject satisfies the second criterion in the second testing method (individual probability). The calculation may be carried out by statistical analysis or analysis by machine learning (comprising deep learning). Also, in an aspect, in the first step, the preliminary method comprising the first step, and the first testing method, it is possible to calculate a probability that a group comprising all subjects subjected to the first step satisfies the second criterion in the second testing method (average of all subjects). In an aspect, in the first step, the preliminary method comprising the first step, and the first testing method, the average of all subjects and the individual probability may be obtained. In an aspect, in the first step, the preliminary method comprising the first step, and the first testing method, it is possible to calculate how high the individual probability is in comparison with the average of all subjects.

The multi-step testing method may further include an additional testing method. The testing methods may each include determining presence or absence of a biomarker in a biological sample obtained from a subject, estimating a health condition of a subject based on an amount of a biomarker, or comparing the amount of the biomarker with a reference value. The reference value is typically associated with a health condition of a subject. For example, presence or absence of a biomarker is associated with a health condition, a risk of developing a disease, responsiveness to a drug, and/or a disease condition of a subject. In an aspect, the testing methods may each provide information for predicting a health condition, a risk of developing a disease, responsiveness to a drug, and/or a disease condition of a subject.

In an aspect, presence of a biomarker is associated with a health condition, a risk of developing a disease, responsiveness to a drug, and/or a disease condition of a subject. In this aspect, absence of a biomarker is associated with a health condition, a risk of developing a disease, responsiveness to a drug, and/or a disease condition of a subject. Such qualitative detection of a biomarker includes, without being particularly limited, for example, a DNA sequence (for example, translocation of DNA, fusion, insertion/deletion (indel), DNA polymorphism, for example, single nucleotide polymorphism (SNIP) of DNA and amplified fragment length polymorphism (AFLP)), immunochromatography, and PCR (particularly nucleic acid amplification other than quantitative PCR).

In an aspect, an amount of a biomarker is associated with a health condition, a risk of developing a disease, responsiveness to a drug, and/or a disease condition of a subject. In this aspect, an amount of a biomarker varies in a subject having a specific disease condition or a disease onset risk. Such quantitative detection of a biomarker includes a quantification method by next-generation sequencing (NGS), a quantification method by digital PCR, a quantification method by mass spectrometry, a quantification method by quantitative PCR, and a quantification method by biochemical test. In quantitative analysis, mRNA and microRNA may be quantified. In quantitative analysis, proteins and others may be quantified. For example, in an aspect, an amount of a biomarker being greater than a reference value and the presence of the biomarker is associated with a health condition, a risk of developing a disease, responsiveness to a drug, and/or a disease condition of a subject. In an aspect, an amount of a biomarker being smaller than a reference value is associated with a health condition, a risk of developing a disease, responsiveness to a drug, and/or a disease condition of a subject. Therefore, in an aspect, it is possible to estimate a health condition, a risk of developing a disease, responsiveness to a drug, and/or a disease condition of a subject based on the amount of the biomarker.

Next-generation sequencing (NGS) is a sequencing technique that increases the throughput of a single sequencing process by performing sequencing reactions in parallel. Some NGS perform millions to billions of sequencing reactions simultaneously in parallel. NGS typically requires preparation of a nucleic acid library to be subjected to the sequencing reaction. The library includes ligating adapters for the sequencing reaction, barcode sequences unique to nucleic acids, and/or index sequences unique to samples, to nucleic acid fragments. The nucleic acids may be amplified (for example, amplified by bridge PCR or emulsion PCR). The amplification products may be, for example, denatured to single strands, and thereafter, the sequences of the fragments may be decoded by DNA synthesis in the presence of dNTPs including fluorescently labeled dNTPs with terminator caps, pyrosequencing, or ligation of fluorescently labeled single-stranded nucleic acids.

Digital PCR is a technique for dispensing template nucleic acids into a large number of wells (or droplets) such that no more than two molecules are contained in one well (or one droplet), amplifying nucleic acids by polymerase chain reaction (PCR), counting the number of wells containing amplified nucleic acids or the number of droplets, and thereby estimating the concentration or the number of molecules of the template present in the specimen.

Quantitative PCR is referred to as quantitative real-time PCR. In quantitative PCR, a reporter dye is used that emits light at an intensity corresponding to the amount of amplification products obtained by the PCR amplification reaction. Reporter dyes include double-stranded DNA binding dyes, primer-binding dyes, and dye-labeled oligonucleotides, which are referred to as probes. Also, a probe (particularly a dual-end-labeled probe) that binds to a specific region of the template existing between primers is also preferably used. The dual-end-labeled probe has a fluorescent dye (for example, 6-FAM) at one end, and a dark quencher (for example, BHQ1 or QQ) at the other end, and when the amplification products increase, it binds to the amplification products, and thereafter, one label (particularly the reporter dye) dissociates from the quencher by the exonuclease activity of DNA polymerase, thereby emitting fluorescence.

When a plurality of biomarkers are present, it is possible to score the presence and/or amount of the biomarkers by weighting of each biomarker. Such a scoring technique includes, without being particularly limited, a technique of weighting Z-scores of the expression levels of each biomarker and accumulating them. The Z-score is a numerical value obtained by subtracting the mean value from the relevant numerical value of a data set and dividing by the standard deviation. The weighting may be achieved by, without being particularly limited, for example, multiplying the Z-score by a coefficient corresponding to the weight, and summing the Z-scores multiplied by the coefficients. By the weighting, increases and decreases of important biomarkers are more strongly reflected in the score, thereby making the comparison between the reference value and the score more rational.

In the multi-step testing method, whether to perform or not to perform the second-stage testing method is determined depending on the results of the first step, the preliminary method comprising the first step, and the first-stage testing method. In an aspect, the second testing method is performed based only on the results of the first step, the preliminary method comprising the first step, and the first-stage testing method (particularly that the level of the first biomarker satisfies the first criterion). In an aspect, after performing the first step, the preliminary method comprising the first step, and the first-stage testing method, no notification such as feedback to the subject or the user is made before performing the second-stage testing method.

In the multi-step testing method, the plurality of subjects serve as examinees, all of them are subjected to the first step, the preliminary method comprising the first step, and the first-stage testing method, the second-stage testing method is applied to some of the subjects, but the second-stage testing method is not applied to other some of the subjects. The first step, the preliminary method comprising the first step, and the first-stage testing method are performed by themselves for narrowing down the subjects to which the second-stage testing method is to be applied, but when the first-stage testing method clearly indicates that the second-stage testing is unnecessary (for example, when it is judged to be sufficient to understand the condition to be evaluated), the effect of reducing the cost and effort required for the overall testing may be brought about by not performing the second-stage testing method. When the second-stage testing method requires enormous computational power, it is effective to limit the subjects to be subjected to the second-stage testing method, thereby enabling rapid calculation or enabling calculation for a larger number of subjects.

The plurality of subjects may be 2 or more, 3 or more, 4 or more, 5 or more, 6 or more, 7 or more, 8 or more, 9 or more, or 10 or more. The plurality of subjects may also be, without being particularly limited, 10,000 or fewer, 9,000 or fewer, 8,000 or fewer, 7,000 or fewer, 6,000 or fewer, 5,000 or fewer, 4,000 or fewer, 3,000 or fewer, 2,000 or fewer, 1,000 or fewer, 900 or fewer, 800 or fewer, 700 or fewer, 600 or fewer, 500 or fewer, 400 or fewer, 300 or fewer, 200 or fewer, 100 or fewer, 90 or fewer, 80 or fewer, 70 or fewer, 60 or fewer, 50 or fewer, 40 or fewer, 30 or fewer, 20 or fewer, or 10 or fewer. The plurality of subjects may be, without being particularly limited, for example, 2 to 10,000, 3 to 9,000, 4 to 8,000, 5 to 7,000, 6 to 6,000, 7 to 5,000, 8 to 4,000, 9 to 3,000, 10 to 2,000, 11 to 1,000, 12 to 900, 13 to 800, 14 to 700, 15 to 600, 16 to 500, 17 to 400, 18 to 300, 19 to 200, 20 to 100, 30 to 90, 40 to 80, or 50 to 70. The plurality of subjects may include, for example, the number of persons measured in a fixed period (for example, a span such as 1 day, 1 week, 1 month, 3 months, half a year, or 1 year). The plurality of subjects may also include, for example, the number of persons measurable in one run of large-scale testing. For example, the plurality of subjects may include the number of persons measurable in one run of NGS. The plurality of subjects may also include, for example, the number of persons for which results can be provided by one data processing.

The criterion for selection of biological samples to be subjected to the second step is the first criterion. When the measured value of the first biomarker in the first biological sample satisfies the first criterion, the second biological sample obtained from the subject from which the biological sample is derived is subjected to the second step. Also, when the measured value of the first biomarker satisfies the first criterion, the second step is not performed or may not be performed.

In a preferred aspect, the first step is a step of qualitative analysis, and the second step is a step of quantitative analysis. In a preferred aspect, both the first step and the second step are steps of quantitative analysis.

In an aspect of the present disclosure, the first step may include measuring levels of s types {s is a natural number of 1 or more} of the first biomarker contained in each of the biological samples. In an aspect of the present disclosure, the first step may include identifying a subject from which a biological sample in which the measured value (level) of the first biomarker in the biological sample obtained by measurement satisfies the first criterion is derived. In an aspect, the first step may include measuring levels of s types {s is a natural number of 1 or more} of the first biomarker contained in each of the biological samples; and identifying a subject from which a biological sample in which the level of the first biomarker satisfies the first criterion is derived.

s may be, without being particularly limited, for example, a natural number of 1 or more, 2 or more, 3 or more, 4 or more, 5 or more, 6 or more, 7 or more, 8 or more, 9 or more, or 10 or more. s may also be, without being particularly limited, for example, a natural number of 1,000 or fewer, 900 or fewer, 800 or fewer, 700 or fewer, 600 or fewer, 500 or fewer, 400 or fewer, 300 or fewer, 2,000 or fewer, 100 or fewer, 90 or fewer, 80 or fewer, 70 or fewer, 60 or fewer, 50 or fewer, 40 or fewer, 30 or fewer, 20 or fewer, or 10 or fewer. s may further be, without being particularly limited, for example, 1 to 10, 1 to 100, 2 to 10, 2 to 100, 3 to 10, 3 to 100, 4 to 10, 4 to 100, 5 to 10, 5 to 100, 1 to 1,000, 2 to 900, 3 to 800, 4 to 700, 5 to 600, 6 to 500, 7 to 400, 8 to 300, 9 to 200, 10 to 100, 20 to 90, 30 to 80, 40 to 70, or 50 to 60.

In an aspect, the second step comprises measuring levels of t types {t is a natural number of 2 or more, preferably a natural number exceeding s} of the second biomarker contained in each of biological samples obtained from the subject identified in the first step. In an aspect, the second step may include identifying a subject from which a biological sample in which the measured value (level) of the second biomarker in the biological sample obtained by measurement satisfies the second criterion is derived. In an aspect, the second step comprises measuring levels of t types {t is a natural number of 2 or more, preferably a natural number exceeding s} of the second biomarker contained in each of biological samples obtained from the subject identified in the first step; and identifying a biological sample in which the measured level satisfies the second criterion.

t may be, without being particularly limited, for example, a natural number of 1 or more, 2 or more, 3 or more, 4 or more, 5 or more, 6 or more, 7 or more, 8 or more, 9 or more, 10 or more, 20 or more, 30 or more, 40 or more, 50 or more, 60 or more, 70 or more, 80 or more, 90 or more, 100 or more, 200 or more, 300 or more, 400 or more, 500 or more, 600 or more, 700 or more, 800 or more, 900 or more, or 1,000 or more. t may also be, without being particularly limited, for example, a natural number of 1,000,000 or fewer, 100,000 or fewer, 10,000 or fewer, 1,000 or fewer, 900 or fewer, 800 or fewer, 700 or fewer, 600 or fewer, 500 or fewer, 400 or fewer, 300 or fewer, 2,000 or fewer, 100 or fewer, 90 or fewer, 80 or fewer, 70 or fewer, 60 or fewer, 50 or fewer, 40 or fewer, 30 or fewer, 20 or fewer, or 10 or fewer. t may further be, without being particularly limited, for example, 1 to 1,000,000, 1 to 100,000, 1 to 10,000, 1 to 1,000, 2 to 900, 3 to 800, 4 to 700, 5 to 600, 6 to 500, 7 to 400, 8 to 300, 9 to 200, 10 to 100, 20 to 90, 30 to 80, 40 to 70, or 50 to 60. The second step may be omics analysis (such as proteome analysis, transcriptome analysis, exome analysis, epigenome analysis, and omics analysis of microRNA). In this case, there may be no specific upper limit or lower limit for the number of markers.

In an aspect, t is a natural number greater than s, and t may be a natural number 2 times or more, 3 times or more, 4 times or more, 5 times or more, 6 times or more, 7 times or more, 8 times or more, 9 times or more, 10 times or more, 20 times or more, 30 times or more, 40 times or more, 50 times or more, 60 times or more, 70 times or more, 80 times or more, 90 times or more, 100 times or more, 200 times or more, 300 times or more, 400 times or more, 500 times or more, 600 times or more, 700 times or more, 800 times or more, 900 times or more, or 1,000 times or more greater than s.

In the method of the present disclosure, the number of analyses of biological samples in the first step is p, and the number of analyses of biological samples in the second step is q {wherein p and q are natural numbers satisfying p > q}.

In an aspect, the number of biological samples in which the measured level satisfies the second criterion is r, the number of biological samples in which the measured level satisfies the second criterion when all p biological samples are subjected to the second step without performing the first step is r', and r/q is greater than r'/p,
a biological sample obtained from a subject from which a biological sample in which the measured level does not satisfy the first criterion in the first step is derived is not analyzed in the second step, and thereby, p and q are natural numbers satisfying p > q.

In an aspect, the first step comprises measuring presence or absence of s types {s is a natural number of 1 or more} of the first biomarker contained in each of the biological samples; and identifying a subject from which a biological sample in which presence or absence of the first biomarker satisfies the first criterion is derived.

In a preferred aspect, the first step is a qualitative or quantitative analysis, and the second step is a quantitative analysis. In a preferred aspect, the first step comprises qualitative analysis or quantitative analysis of amplification products of the first biomarker by a PCR method (for example, a nucleic acid array (for example, may be qualitative or quantitative analysis by a DNA array, qualitative analysis by PCR, quantitative analysis by real-time PCR (RT-PCR), or quantitative analysis by digital PCR)), and the second step is a quantitative analysis. In this aspect, the second step preferably comprises quantitative analysis of the second biomarker by sequencing or mass spectrometry of the second biomarker. In this aspect, the first step may include, for example, detecting presence or absence of amplification products of the first biomarker; and determining whether presence or absence of amplification products of the first biomarker satisfies the first criterion. In this aspect, the first step may include, for example, detecting presence or absence of amplification products of the first biomarker; and determining whether the amount of amplification products of the first biomarker satisfies the first criterion. Also, in this aspect, quantitative analysis by sequencing may typically be achieved by ligating a barcode unique to each nucleic acid molecule, and estimating the types of decoded barcodes as the number of nucleic acid molecules.

As the number of second biomarkers increases, the burden or cost of qualitative analysis (for example, sequencing and mass spectrometry) increases. Therefore, in order to reduce the number of biological samples (q) to be subjected to the second step, selection of subjects in the first step is effective. The number of second biomarkers (t) may be, for example, 20 or more, 30 or more, 40 or more, or 50 or more. The definition of t is as described above.

In an aspect, in the above-described second step, a prediction model constructed so as to be able to predict a health condition of a subject from which a biological sample is derived, from a level of the second biomarker in the biological sample, by causing association information between levels of the second biomarker of each of biological samples obtained from a plurality of subjects measured in advance and health conditions of each of the plurality of subjects to be learned by machine learning, includes the second criterion relating to the level of the second biomarker, and the prediction model evaluates whether the level of the second biomarker in the biological sample satisfies the second criterion. In an aspect, the second step or the second testing method infers or predicts a health condition, a risk of developing a disease, responsiveness to a drug, and/or a disease condition of a subject from which the biological sample is derived, based on whether the level of the second biomarker in the biological sample satisfies the second criterion. For example, when the level of the second biomarker in the biological sample satisfies (or does not satisfy) the second criterion, it may be inferred or predicted that the subject from which the biological sample is derived is healthy, has a risk of developing a disease (specific disease), and/or has a disease (specific disease). For example, when the level of the second biomarker in the biological sample does not satisfy (or satisfies) the second criterion, it may be inferred or predicted that the subject from which the biological sample is derived is not healthy, does not have a risk of developing a disease (specific disease), and/or does not have a disease (specific disease).

In an aspect, the method of the present disclosure may further include one or more correction cycles of the first criterion. The correction cycle of the first criterion comprises: associating the level of the first biomarker and the level of the second biomarker of each of the subjects;
causing the associated level of the first biomarker and the level of the second biomarker to be learned by machine learning, and constructing a prediction model capable of predicting the level of the second biomarker from the level of the first biomarker;
wherein the number of biological samples in which the measured level satisfies the second criterion is r {r is a natural number equal to or less than q};
correcting the first criterion so that r becomes larger based on the prediction model without increasing q (that is, while q is constant or preferably decreasing).
Thereby, it becomes possible to more effectively reduce the number of tests (q) in the second testing method comprising the second step, or to more effectively increase r/q (particularly as compared to r/q when no correction cycle is included), in the first step, the preliminary method comprising the first step, and the first testing method.

In this manner, according to the method of the present disclosure, it becomes possible to reduce the number of biological samples (q) to be subjected to the second step, by performing the first step, the preliminary method comprising the first step, or the first testing method comprising the first step, before the second step or the second testing method comprising the second step.

### <System of the Present Disclosure>

According to the present disclosure, a system for testing a plurality of biological samples respectively obtained from a plurality of subjects is provided. The system 100 of the present disclosure has one or more processors 105, a memory 104, and an output device, and one or more programs are stored in the memory 104 and configured to be executed by the one or more processors 105.

The one or more programs are configured to execute, for example, the above-described method of the present disclosure.

In a preferred aspect of the present disclosure, the one or more programs comprise instructions for:
causing the memory to store (preferably, ID information for identifying a subject and) levels of s types {s is a natural number of 1 or more} of a first biomarker contained in each of biological samples obtained from the subjects (number of individuals: p) and a first criterion, causing the processor to calculate whether the level of the first biomarker stored in the memory falls under the first criterion, and (preferably, based on the ID information) causing the processor to identify subjects (number of individuals: q) from which biological samples in which the level of the first biomarker falls under the first criterion are derived, and causing the output device to output the subjects (first step); and
causing the memory to store (preferably, ID information for identifying the subject and) levels of t types {t is a natural number of 1 or more} of a second biomarker contained in each of biological samples obtained from the subjects (number of individuals: q) output by the output device and a second criterion, causing the processor to calculate whether the level of the second biomarker stored in the memory falls under the second criterion, and (preferably, based on the ID information) causing the processor to identify subjects (number of individuals: r) from which biological samples in which the level of the second biomarker falls under the second criterion are derived, and causing the output device to output the subjects (second step).

The ID information need only be information that identifies a subject person, and may be, for example, the real name of the subject person, or may be character information (for example, which may include numbers, symbols, uppercase letters, and lowercase letters).

In a preferred aspect, p, q, and r are each natural numbers, p > q > r, the number of biological samples in which the measured level satisfies the second criterion when all p biological samples are subjected to the second step is r', and r/q is greater than r'/p.

In the above, the output device 101 may be, for example, a display, a printer, or a speaker. The output device 101 may be provided in the system 100 of the present disclosure, or may not be provided in the system of the present disclosure. For example, the output device 101 may be a terminal such as a smartphone or a tablet, and the processor may be configured to transmit a command to the terminal and cause the subject to be displayed (output) on the screen of the terminal. In transmitting a command from the system 100 to the terminal, it may be via the communication interface 107. The communication interface 107 may transmit and receive radio waves such as Wi-Fi or Bluetooth. The communication interface 107 has a transceiver for universal serial bus (USB) or local area network (LAN), and may transmit a command to the terminal via a local area network or the Internet to display information on the screen of the terminal.

The system 100 of the present disclosure may further include an input device 102 and/or a cursor control device 103. The input device 102 may be, for example, a keyboard or a microphone. The cursor control device 103 may be, for example, a mouse.

In the system 100 of the present disclosure, the processor 105, the memory 104, the input device 102, the cursor control device 103, and the output device 101 may be operably connected via a bus 108. The system 100 of the present disclosure may further have a communication interface 107, and the communication interface 107 may also be operably connected to other components of the system 100 of the present disclosure via the bus 108. The communication interface 107 may also be communicably connected to a local network by wire. The local network may be communicably connected to a terminal of the user via the Internet.

In an aspect, in the system of the present disclosure,
a time required for a program executing the first step and the second step is shorter than a time required for executing a control program,
the control program comprising instructions for causing the memory to store levels of t types {t is a natural number of 1 or more} of a second biomarker contained in each of biological samples obtained from subjects (number of individuals: p) and a second criterion, causing the processor to calculate whether the level of the second biomarker stored in the memory falls under the second criterion, and causing the processor to identify subjects (number of individuals: r) from which biological samples in which the level of the second biomarker falls under the second criterion are derived, and causing the output device to output the subjects. When the number of second biomarkers increases or the amount of calculation for determining whether they fall under the second criterion is enormous, it is particularly beneficial that the time required for the program executing the first step and the second step is shorter than the time required for executing the control program.

In an aspect, the program further comprises instructions for executing one or more correction cycles of the first criterion,
the correction cycle of the first criterion comprising:
associating the level of the first biomarker and the level of the second biomarker of each of the subjects;
causing the associated level of the first biomarker and the level of the second biomarker to be learned by machine learning, and constructing a prediction model capable of predicting the level of the second biomarker from the level of the first biomarker;
wherein the number of biological samples in which the measured level satisfies the second criterion is r {r is a natural number equal to or less than q};
correcting the first criterion so that r becomes larger based on the prediction model without increasing q (that is, while q is constant or preferably decreasing).
Thereby, it becomes possible to more effectively reduce the number of tests (q) in the second testing method comprising the second step, or to more effectively increase r/q (particularly as compared to r/q when no correction cycle is included), in the first step, the preliminary method comprising the first step, and the first testing method.

In an aspect, in the program, the first step can calculate a probability that the subject satisfies the second criterion in the second step (individual probability) based on the level of the first biomarker. The calculation may be carried out by statistical analysis or analysis using a trained model by machine learning (including deep learning). Also, in an aspect, in the program, in the first step, it is possible to calculate a probability that a group including all subjects subjected to the first step satisfies the second criterion in the second testing method (average of all subjects). In an aspect, in the program, in the first step, the average of all subjects and the individual probability are obtained. In an aspect, in the first step, the preliminary method comprising the first step, and the first testing method, it is possible to calculate how high the individual probability is in comparison with the average of all subjects. In a preferred aspect, the program displays information recommending the performance of the second testing method to the user, or transmits it to the terminal of the user, in the first step.

In a preferred aspect, the information recommending the performance of the second testing method can recommend the performance of the second testing method to the user at an intensity corresponding to the individual probability. The program can indicate the level of necessity of performing the second testing method to the user at an intensity corresponding to the individual probability, such as, for example, presenting the individual probability, the degree of deviation of the individual probability from the average of all subjects, and the scored individual probability to the user.

In a preferred aspect, the program may include a trained model obtained by machine learning using information on whether the subject satisfies the second criterion in the second step and information on the level of the first biomarker as training data. The program comprising the trained model can calculate a probability that the subject satisfies the second criterion in the second step (individual probability) based on the level of the first biomarker. The program can indicate the level of necessity of performing the second testing method to the user at an intensity corresponding to the individual probability calculated from the above-described trained model, for example.

The information recommending the performance of the second testing method is preferably displayed on a display, but more preferably printed on paper and sent to the user.

As described above, the system of the present disclosure is beneficial in performing the method of the present disclosure.

### <Billing System>

In order to realize the multi-step testing method of the present disclosure, a billing method suitable for the characteristics of the testing method is necessary. For example, in the multi-step testing method, there are subjects to which only the first step is applied (subject A) and subjects to which the second step is applied in addition to the first step (subject B), and it is necessary to address the issue of whether both subject A and B should be billed the same fee or should be billed different fees.

In an aspect, the billing program provides electronic payment means for receiving payment of a uniform fee for the first step and the second step from all users. The merit of both subject A and B paying the same fee is, for example, that when the second step is very expensive and q is extremely small relative to p, by making the fee uniform for all, the burden on subject B who undergoes the second step can be reduced. This billing method is similar to insurance. That is, many subjects can complete the testing with only the first step, but when some subjects unfortunately need to undergo the second step, they can undergo the testing of the second step without additional burden for the second step. For example, when q is 1/5 or less, 1/10 or less, more preferably 1/50 or less, 1/100 or less, still more preferably 1/500 or less, or 1/1,000 or less of p, by paying the cost for subject B equally by all, the burden on subject B is greatly reduced by a limited increase in burden for each user. The smaller q/p is, the more limited the increase in burden for each user. In an aspect, the list price of the first step may be 1/5 or less, 1/10 or less, 1/50 or less, 1/100 or less, 1/500 or less, or 1/1,000 or less of the list price of the second step.

In an aspect, the billing program provides electronic payment means for receiving payment of a uniform fee for the first step and the second step from all users. In this aspect, the billing program issues a discount coupon for the next multi-step testing method to the account of the user when it is determined that the subject will not undergo the second step in the first step, the preliminary method comprising the first step, and the first testing method. This billing method provides a merit to subject A, for example, when the financial demerit of subject A not undergoing the second step is large.

In an aspect, the billing program does not require payment from the user for the first step, the preliminary method comprising the first step, and the first testing method. In this aspect, the billing program provides electronic payment means for payment to the user regarding the use of the second step by the subject. This billing method may be beneficial in increasing the number of users for the first step, the preliminary method comprising the first step, and the first testing method, thereby increasing the potential users of the second step. For example, it is a billing method that is beneficial when it is difficult not to receive the application of the second step, or when it is difficult to take the option of not receiving the application of the second step, in the first step, the preliminary method comprising the first step, and the first testing method.

When the user of the second step is using the first step, the preliminary method comprising the first step, and the first testing method, the fee regarding the use of the second testing method may be reduced. By doing so, it is possible to increase the number of users who enjoy the series of steps including the first step and the second step as a set. In an aspect, the billing program may propose a fee reduction for the use of the second testing method or issue a discount coupon to the account of the user who has made payment for the first step, the preliminary method comprising the first step, and the first testing method. The discount on the fee regarding the use of the second testing method may be beneficial, for example, when the biomarkers of the first step and the second step overlap, and the testing in the second step can be simplified by using the results of the first step. Also, it may encourage the use of the series of steps comprising the first step and the second step as a set for users who would otherwise only undergo one of the series of steps comprising the first step and the second step. The discount amount on the fee regarding the use of the second testing method may be the same as or less than the fee regarding the first step, the preliminary method comprising the first step, and the first testing method.

When the user of the second step is using the first step, the preliminary method comprising the first step, and the first testing method, the program may calculate the individual probability in the first step and change (for example, reduce or increase) the fee regarding the use of the second testing method in accordance with the level of the individual probability. In a preferred aspect, when the individual probability is higher, the fee regarding the use of the second testing method can be reduced. Users with a high individual probability may have reduced motivation to perform the second testing method. However, by reducing the fee when the individual probability is high, the performance of the second testing method by users who have learned of the high individual probability may be promoted. In a preferred aspect, when the individual probability is lower, the fee regarding the use of the second testing method can be reduced. Users who have learned of the low individual probability may have reduced motivation to perform the second testing method. However, by reducing the fee when the individual probability is high, the performance of the second testing method by users who have learned of the low individual probability may be promoted. In an aspect, the more the individual probability deviates from the average of all subjects, the more the fee regarding the use of the second testing method can be reduced. Thereby, the performance of the second testing method by users may be promoted for any of the above cases.

The above is merely an example, but the billing program of the present disclosure may fulfill an insurance-like function of reducing excessive burden on users, or may maximize revenue from the performance of the method of the present disclosure.

In an aspect, the billing program provides electronic payment means for payment from all users of the first step, the preliminary method comprising the first step, and the first testing method. The billing program also provides electronic payment means for payment to users of the second step by the subject.

In a preferred aspect, the one or more programs may further cause the one or more processors to execute a billing program. The billing program provides electronic payment means to the user. The electronic payment means may include one payment method or a plurality of selectable payment methods. The payment method may be, for example, electronic payment such as deferred payment or credit card payment. The payment method may also be one in which payment is made from the balance of electronic money charged to the account of the user.

## Claims

1. A method for testing a plurality of biological samples respectively obtained from a plurality of subjects,
the method comprising a first step and a second step, the second step being performed after the first step,
the first step comprising:
measuring levels of s types {s is a natural number of 1 or more} of a first biomarker contained in each of the biological samples; and
identifying a subject from which a biological sample in which a level of the first biomarker satisfies a first criterion is derived,
the second step comprising:
measuring levels of t types {t is a natural number of 2 or more, preferably a natural number exceeding s} of a second biomarker contained in each of biological samples obtained from the subject identified in the first step; and
identifying a biological sample in which the measured level satisfies a second criterion,
wherein the number of analyses of biological samples in the first step is p,
the number of analyses of biological samples in the second step is q,
the number of biological samples in which the measured level satisfies the second criterion is r, the number of biological samples in which the measured level satisfies the second criterion when all p biological samples are subjected to the second step without performing the first step is r', and r/q is greater than r'/p,
a biological sample obtained from a subject from which a biological sample in which the measured level does not satisfy the first criterion in the first step is derived is not analyzed in the second step,
and thereby, p and q are natural numbers satisfying p > q.

2. The method according to Claim 1, wherein
the first step comprises measuring presence or absence of s types {s is a natural number of 1 or
more} of the first biomarker contained in each of the biological samples; and identifying a subject from which a biological sample in which presence or absence of the first biomarker satisfies the first criterion is derived.

3. The method according to Claim 1 or 2, wherein
the level of the first biomarker is measured by a DNA array or a PCR method, and
the level of the second biomarker is measured by a sequencing method.

4. The method according to Claim 3, wherein the PCR method is a digital PCR method.

5. The method according to Claim 3 or 4, wherein t is a natural number of 20 or more.

6. The method according to any one of Claims 1 to 5, wherein
in the second step, a prediction model constructed so as to be able to predict a health condition of a subject from which a biological sample is derived, from a level of the second biomarker in the biological sample, by causing association information between levels of the second biomarker of each of biological samples obtained from a plurality of subjects measured in advance and health conditions of each of the plurality of subjects to be learned by machine learning, includes the second criterion relating to the level of the second biomarker, and the prediction model evaluates whether the level of the second biomarker in the biological sample satisfies the second criterion.

7. The method according to any one of Claims 1 to 6, further comprising one or more correction cycles of the first criterion,
the correction cycle of the first criterion comprising:
associating the level of the first biomarker and the level of the second biomarker of each of the subjects;
causing the associated level of the first biomarker and the level of the second biomarker to be learned by machine learning, and constructing a prediction model capable of predicting the level of the second biomarker from the level of the first biomarker;
wherein the number of biological samples in which the measured level satisfies the second criterion is r {r is a natural number equal to or less than q}; and
correcting the first criterion so that r becomes larger based on the prediction model without increasing q (that is, while q is constant or preferably decreasing).

8. A system for testing a plurality of biological samples respectively obtained from a plurality of subjects,
having one or more processors, a memory, and an output device,
one or more programs being stored in the memory and configured to be executed by the one or more processors,
the one or more programs comprising instructions for:
causing the memory to store levels of s types {s is a natural number of 1 or more} of a first biomarker contained in each of biological samples obtained from the subjects (number of individuals: p) and a first criterion, causing the processor to calculate whether the level of the first biomarker stored in the memory falls under the first criterion, and causing the processor to identify subjects (number of individuals: q) from which biological samples in which the level of the first biomarker falls under the first criterion are derived, and causing the output device to output the subjects (a first step); and
causing the memory to store levels of t types {t is a natural number of 1 or more} of a second biomarker contained in each of biological samples obtained from the subjects (number of individuals: q) output by the output device and a second criterion, causing the processor to calculate whether the level of the second biomarker stored in the memory falls under the second criterion, and causing the processor to identify subjects (number of individuals: r) from which biological samples in which the level of the second biomarker falls under the second criterion are derived, and causing the output device to output the subjects (a second step),
wherein p, q, and r are each natural numbers, p > q > r, the number of biological samples in which the measured level satisfies the second criterion when all p biological samples are subjected to the second step is r', and r/q is greater than r'/p.

9. The system according to Claim 8, wherein
a time required for a program executing the first step and the second step is shorter than a time required for executing a control program,
the control program comprising instructions for causing the memory to store levels of t types {t is a natural number of 1 or more} of the second biomarker contained in each of biological samples obtained from subjects (number of individuals: p) and the second criterion, causing the processor to calculate whether the level of the second biomarker stored in the memory falls under the second criterion, and causing the processor to identify subjects (number of individuals: r) from which biological samples in which the level of the second biomarker falls under the second criterion are derived, and causing the output device to output the subjects.

10. The system according to Claim 8 or 9, wherein
the program further comprises instructions for executing one or more correction cycles of the first criterion,
the correction cycle of the first criterion comprising:
associating the level of the first biomarker and the level of the second biomarker of each of the subjects;
causing the associated level of the first biomarker and the level of the second biomarker to be learned by machine learning, and constructing a prediction model capable of predicting the level of the second biomarker from the level of the first biomarker;
wherein the number of biological samples in which the measured level satisfies the second criterion is r {r is a natural number equal to or less than q}; and
correcting the first criterion so that r becomes larger based on the prediction model without increasing q (that is, while q is constant or preferably decreasing).
